# EUROPEAN PATENT SPECIFICATION

(11) **EP 2 508 216 B1**
(45) Date of publication and mention of the grant of the patent: **06.09.2017**
(21) Application number: 12162932.3
(22) Date of filing: 03.04.2012
(51) Int. Cl.: A61M 5/148, A61M 5/32, A61M 5/28, A61M 5/34

(54) **Medicine delivery system**
Medikamentenabgabesystem
Système d'administration de médicament

(30) Priority: 05.04.2011 US 201161471916 P
(43) Date of publication of application: 10.10.2012
(73) Proprietor: Covidien LP, Mansfield, MA 02048 (US)
(72) Inventor: Brundidge III, Edward, Cumberland, Rhode Island 02864 (US); Callahan, Mark J., Medway, Massachusetts (US); Delnickas, Jeffrey T., Kingston, Massachusetts 02364 (US); Elavia, Teshtar A., Weymouth, Massachusetts 02188 (US); Sharp, Brian P., Johnston, Rhode Island 02919 (US)
(74) Representative: Gray, James

(56) References cited:
- WO-A1-94/22509
- DE-A1- 3 106 382
- GB-A- 1 083 335
- US-A- 3 989 045
- US-A- 4 475 906
- US-A- 4 522 622
- US-A1- 2003 014 019

## Description

### CROSS REFERENCE TO RELATED APPLCIATION

This application claims priority to U.S. Patent Application 61/471,916 filed April 5, 2011, which is hereby incorporated by reference in its entirety.

### BACKGROUND

The present invention generally relates generally to devices for injecting liquid medications, and more particularly to a bulb driven injection system. Examples of existing systems are described in DE 3106382, US 4475906, GB 1083335, US 3989045 and WO94/22509.

Local anesthetic is frequently used to numb tissue in a patient's mouth to reduce pain and discomfort a patient may feel during a dental procedure. Conventionally, a reusable syringe assembly is used to inject the anesthetic or medicine from a cartridge or carpule. The cartridge is a glass cylinder containing a local anesthetic and other ingredients. A diaphragm at one end of the cylinder is held in place by an aluminum band. The opposite end of the cylinder has a moveable piston or stopper. The syringe assembly includes a barrel for receiving the cartridge, a plunger rod slidably received in a proximal end of the barrel for actuating the cartridge, an access needle at a distal end of the barrel for puncturing the diaphragm, and a delivery needle connected to the access needle for delivering anesthetic to the patient. In some cases, the plunger rod includes a harpoon for engaging the piston.

Typically, the diaphragm of the cartridge is swabbed with alcohol before being loaded into a pre-sterilized syringe. As the cartridge is loaded into the syringe, the access needle extending proximally from the distal end of the barrel pierces the cartridge diaphragm so the anesthetic in the cartridge can be dispensed. Once the cartridge is in place, the plunger rod of the syringe pushes the piston of the cartridge toward the diaphragm, forcing anesthetic through the access needle, into the delivery needle, and ultimately into the patient.

Conventional syringe systems can be imposing devices that cause anxiety for many patients. Thus, there is a need for an injection device having a novel shape that is less recognizable for patients so they are less apt to become anxious. Further, many conventional syringes have long plunger travel distances that require the user to have large hands to operate. Thus, there is a need for an injection system permitting users to have smaller hands, and more preferably to adjust to the size of the user's hands.

### SUMMARY

The present invention relates to an injection assembly for injecting medicine according to claim 1.

Embodiments of the present invention will be apparent in view of the following description and claims.

### BRIEF DESCRIPTION OF THE DRAWINGS

Fig. 1 is a perspective of an injection assembly of a first embodiment of the present invention;
Fig. 2 is a perspective of an injection assembly of a second embodiment of the present invention; and
Fig. 3 is a perspective of an injection assembly of a third embodiment of the present invention.
Corresponding reference characters indicate corresponding parts throughout the drawings.

### DETAILED DESCRIPTION OF THE DRAWINGS

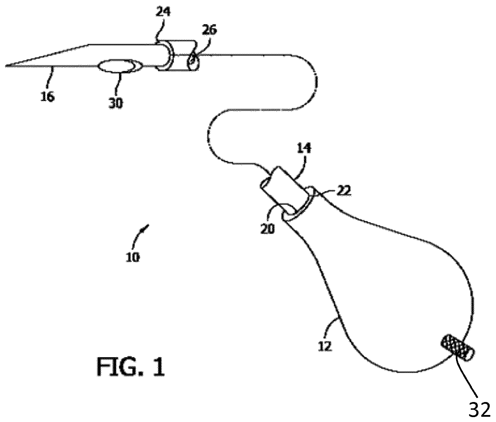

Referring to Fig. 1, an injection assembly of a first embodiment of the present invention is generally designated in its entirety by the reference number 10. The syringe assembly 10 includes a compressible reservoir, such as a collapsible elastomeric bulb 12, a tube 14, and a needle 16. The collapsible elastomeric bulb has a hollow interior (not shown) and an outlet 20 extending from the interior. The bulb is collapsible from an expanded configuration shown in Fig. 1 in which the hollow interior has an expanded volume to a collapsed configuration in which the hollow interior has a collapsed volume that is smaller than the expanded volume. As will be apparent to those skilled in the art, collapsing the elastomeric bulb 12 forces fluid from the hollow interior of the bulb through its outlet 20. The transparent flexible tube 14 extends from the outlet 20. The tube 14 has a proximal end 22 connected to the bulb 12 and a distal end 24 opposite the proximal end. An interior passage 26 of the tube 14 is in fluid communication with the outlet 20 of the bulb 12. Thus, fluid exiting the bulb 12 through the outlet 20 enters the interior passage 26 of the tube 14 and flows toward the needle 16. The needle 16 is connected to the distal end 24 of the tube 14 and has an interior passage (not shown) in fluid communication with the distal end of the tube. The needle 16 delivers medicine such as anesthetic from the hollow interior of the bulb 12 when the bulb is compressed toward the collapsed configuration. When the bulb 12 is permitted to expand, the needle 16 can draw fluid toward the bulb to aspirate the region. It is envisioned that the bulb 12 may have any conventional means for promoting its expansion including its own resilience.

As further shown in Fig. 1, the needle 16 may include a receiver 30 for receiving standard dental tools (not shown). Although the needle 16 may have other configurations without departing from the scope of the present invention, in one embodiment the needle is a small gauge short needle and is replaceable. The bulb 16 may also include an adjustment valve 32 to allow fluid into or out of the bulb thereby changing the size of the bulb to fit different sized hands.

A seal or valve (not shown) may be positioned at the distal end of the bulb 12 to prevent fluid exiting the reservoir prior to or after use. Alternatively, a seal or valve may be positioned between the bulb 12 and the flexible tube 14. The seal or valve may be opened when sufficient pressure is exerted on the flexible bulb. The seal may be a split septum or thin film that ruptures when loaded to a predetermined pressure. The valve may be a one or two way valve and be configured to be activated manually or automatically under a predetermined pressure.

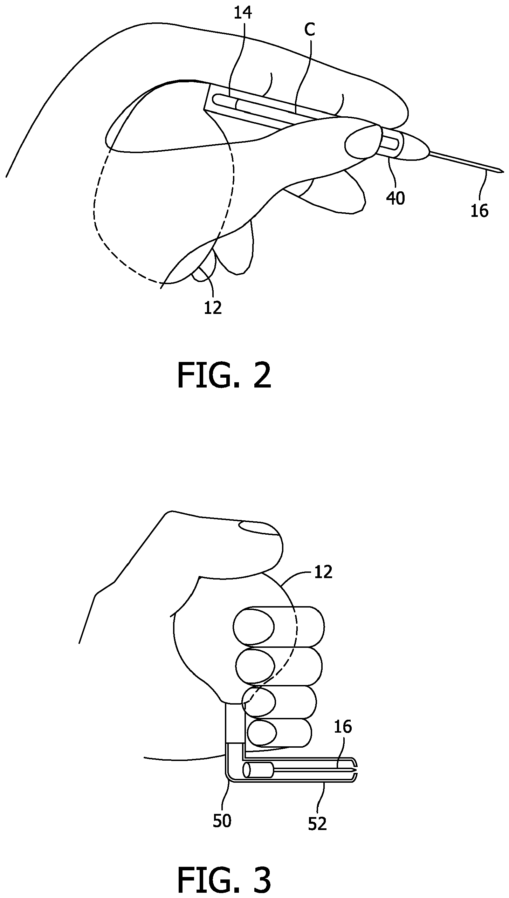

In a second embodiment, shown in Fig. 2, the flexible tube 14 is housed in a pencil sized tube 40 containing a cartridge C. The fluid pressure from the bulb 12 is used to drive a stopper of the cartridge C to force fluid from the cartridge and through the needle 16. Those skilled in the art will appreciate other details of this embodiment.

In a third embodiment shown in Fig. 3, the flexible tube 14 is replaced with an L-shaped rigid tube 50. The bulb 12 can be held in a user's fist so the user's thumb engages the bulb. In this position, the thumb can be used to squeeze the bulb 12. It is envisioned this embodiment could include a sheath 52 that covers the needle 16 until inside the patient's mouth. In one embodiment, increasing pressure of the fluid causes the needle 16 to advance out of the sheath 52 where it can be used in a conventional manner. A seal or valve, as described above, may also be provided to prevent fluid from exiting the fluid reservoir in the bulb prior to or after use.

It is believed that these anesthetic systems described above may be less intimidating to patients than current aspirating syringes, potentially reducing anxiety and fear. Toward that end, in some embodiments the aforementioned devices may be produced in colors matching surgical gloves to further disguise their presence. Further, it is believed that using collapsible elastomeric bulbs may minimize patient pain associated with tissue expansion during administration of the local anesthetic by allowing the clinician to more carefully deliver the fluid to the tissue thereby reducing tissue expansion and the pain associated with tissue expansion. In addition, the bulbs can provide smooth and continuous flow of anesthetic to minimize the patient's pain due to fluctuations in anesthetic flow.

It should be noted that any of the features described hereinabove may be combined with one or more of any of the other features described herein.

While several embodiments of the disclosure have been shown in the drawings and/or discussed herein, it is not intended that the disclosure be limited thereto, as it is intended that the disclosure be as broad in scope as the art will allow and that the specification be read likewise. Therefore, the above description should not be construed as limiting, but merely as exemplifications of particular embodiments. The claims can encompass embodiments in hardware, software, or a combination thereof. Those skilled in the art will envision other modifications within the scope and spirit of the claims appended hereto.

Having described the invention in detail, it will be apparent that modifications and variations are possible without departing from the scope of the invention defined in the appended claims.

When introducing elements of the present invention or the preferred embodiment(s) thereof, the articles "a", "an", "the", and "said" are intended to mean that there are one or more of the elements. The terms "comprising", "including", and "having" are intended to be inclusive and mean that there may be additional elements other than the listed elements.

As various changes could be made in the above constructions, products, and methods without departing from the scope of the invention, it is intended that all matter contained in the above description and shown in the accompanying drawings shall be interpreted as illustrative and not in a limiting sense.

## Claims

1. An injection (10) assembly for injecting medicine, the assembly comprising:
a selectively collapsible elastomeric bulb (12) having a hollow interior and an outlet (20) extending from the interior, the bulb (12) being collapsible from an expanded configuration in which the hollow interior has an expanded volume to a collapsed configuration in which the hollow interior has a collapsed volume that is smaller than the expanded volume;
a tube (14) having a proximal end (22) connected to bulb (12), a distal end (24) opposite the proximal end (22), and an interior passage (26) extending from the proximal end (22) to the distal end (24) of the tube (14) in fluid communication with the outlet (20) of the bulb (12); and
a needle (16) connected to the distal end (24) of the tube (14) and having a sharp distal end, the needle (16) having an interior passage in fluid communication with the interior passage (26) of the tube (14) for delivering medicine from the hollow interior of the bulb (12) when the bulb (12) is compressed toward the collapsed configuration and drawing fluid through the needle (16) toward the bulb (12) when the bulb (12) expands toward the expanded configuration, **characterised in that** the needle (16) includes a receiver (30) for receiving a dental tool therein.

2. An assembly (10) as set forth in claim 1, wherein the tube comprises a flexible tube.

3. An assembly (10) as set forth in claim 1, wherein the bulb (12) further comprises an adjustment valve (32) to allow fluid into or out of the bulb thereby changing the size of the bulb.

4. An assembly (10) as set forth in claim 1, wherein the bulb (12) is pre-filled with an anesthetic solution.

## Patentansprüche

1. Injektionsanordnung (10) zur Injektion von Medizin, wobei die Anordnung Folgendes umfasst:
ein selektiv zusammenlegbarer Elastomerdruckball (12) mit einem hohlen Innenraum und einem Ausgang (20), der sich von dem Innenraum erstreckt, wobei der Druckball (12) von einer expandierten Konfiguration, in welcher der hohle Innenraum ein expandiertes Volumen aufweist, in eine zusammengelegte Konfiguration, in welcher der hohle Innenraum ein zusammengelegtes Volumen aufweist, das geringer ist als das expandierte Volumen, zusammenlegen lässt;
einen Schlauch (14) mit einem proximalen Ende (22), das mit dem Druckball (12) verbunden ist, einem distalen Ende (24) gegenüber dem proximalen Ende (22) und einem Innendurchgang (26), der sich von dem proximalen Ende (22) bis zu dem distalen Ende (24) des Schlauchs (14) in Fluidkommunikation mit dem Ausgang (20) des Druckballs (12) erstreckt; und
eine Nadel (16), die mit dem distalen Ende (24) des Schlauchs (14) verbunden ist und ein spitzes distales Ende aufweist, wobei die Nadel (16) einen Innendurchgang in Fluidkommunikation mit dem Innendurchgang (26) des Schlauchs (14) zur Abgabe von Medizin aus dem hohlen Innenraum des Druckballs (12) aufweist, wenn der Druckball (12) zu der zusammengelegten Konfiguration hin zusammengedrückt wird und zum Abnehmen von Fluid durch die Nadel (16) zum Druckball (12), wenn sich der Druckball (12) zu der expandierten Konfiguration hin expandiert, **dadurch gekennzeichnet, dass** die Nadel (16) einen Empfänger (30) zur Aufnahme eines zahnärztlichen Instruments darin enthält.

2. Anordnung (10) nach Anspruch 1, wobei der Schlauch einen flexiblen Schlauch umfasst.

3. Anordnung (10) nach Anspruch 1, wobei der Druckball (12) ferner ein Stellventil (32) umfasst, um Fluid in den Druckball hinein- oder aus dem Druckball herauszulassen, wodurch die Größe des Druckballs verändert wird.

4. Anordnung (10) nach Anspruch 1, wobei der Druckball (12) mit einer Betäubungsmittellösung vorgefüllt ist.

## Revendications

1. Système d'administration de médicament (10) pour injecter un médicament, le système comprenant :une ampoule en élastomère qui peut être sélectivement affaissée (12) ayant un intérieur creux et une sortie (20) se prolongeant de l'intérieur, l'ampoule (12) pouvant être affaissée d'une position expansée dans laquelle l'intérieur creux possède un volume expansé vers une configuration affaissée dans laquelle l'intérieur creux possède un volume affaissé qui est inférieur au volume expansé ;
un tube (14) ayant une extrémité proximale (22) reliée à l'ampoule (12), une extrémité distale (24) à l'opposé de l'extrémité proximale (22), et un passage intérieur (26) se prolongeant de l'extrémité proximale (22) vers l'extrémité distale (24) du tube (14) en communication fluide avec la sortie (20) de l'ampoule (12) ; etune aiguille (16) reliée à l'extrémité distale (24) du tube (14) et ayant une extrémité distale pointue, l'aiguille (16) possédant un passage interne en communication fluide avec le passage interne (26) du tube (14) pour administrer un médicament à partir de l'intérieur creux de l'ampoule (12) lorsque l'ampoule (12) est comprimée vers la configuration affaissée et l'aspiration de fluide à travers l'aiguille (16) vers l'ampoule (12) lorsque l'ampoule (12) se dilate vers la configuration expansée, **caractérisée en ce que** l'aiguille (16) comprend un récepteur (30) pour recevoir un outil dentaire dans celui-ci.

2. Système (10) selon la revendication 1, dans lequel le tube comprend un tube flexible.

3. Système (10) selon la revendication 1, dans lequel l'ampoule (12) comprend également une valve d'ajustement (32) pour laisser passer un fluide dans et hors de l'ampoule modifiant ainsi la taille de l'ampoule.

4. Système (10) selon la revendication 1, dans lequel l'ampoule (12) est pré-remplie d'une solution anesthésiante.
